# FASCICULE DE BREVET EUROPEEN

(11) **EP 1 763 355 B1**
(45) Date de publication et mention de la délivrance du brevet: **06.11.2019**
(21) Numéro de dépôt: 05763732.4
(22) Date de dépôt: 29.04.2005
(51) Int. Cl.: A61K 31/7004, A61K 36/48, A61K 8/60, A61K 8/97, A61Q 19/00, A61P 17/00, A61P 17/02, A61P 17/06, A61P 31/00, A61P 17/08, A61P 17/10, A61P 19/02

(54) **Composition comprenant du D-mannoheptulose et/ou perséitol pour le traitement ou la prévention de maladies liées à une modification de l'immunité innée et/ou acquise**
Zusammensetzung enthaltend D-Mannoheptulose und/oder Perseitol zur Behandlung oder Vorbeugung von Krankheiten, die mit einer Veränderung der angeborenen und/oder erworbenen Immunität verbunden sind
Composition comprising D-mannoheptulose and/or perseitol for the treatment or prevention of diseases linked to a modification of the innate and/or acquired immunity

(30) Priorité: 30.04.2004 FR 0404635
(43) Date de publication de la demande: 21.03.2007
(73) Titulaire: Laboratoires Expanscience, 92048 Paris La Défense Cedex (FR)
(72) Inventeur: PICCIRILLI, Antoine, F-78670 Villennes Sur Seine (FR); PICCARDI, Nathalie, F-21310 Arceau (FR); MSIKA, Philippe, F-78000 Versailles (FR); PAUL, François, F-09160 Montgauch (FR); BREDIF, Stéphanie, F-28210 Chaudon (FR)
(74) Mandataire: Regimbeau
(86) Numéro de dépôt international: PCT/FR2005/001075
(87) Numéro de publication internationale: WO 2005/115421

(56) Documents cités:
- WO-A-01/82889
- WO-A2-01/68040
- FR-A- 2 843 125
- US-A- 4 722 843
- US-A1- 2003 092 669
- GALLAGHER A M ET AL: "The effects of traditional antidiabetic plants on in vitro glucose diffusion" NUTRITION RESEARCH 01 MAR 2003 UNITED STATES, vol. 23, no. 3, 1 mars 2003 (2003-03-01), pages 413-424, XP001205252 ISSN: 0271-5317
- RAONIMALALA A F ET AL: "Effect of soluble carbohydrates from avocado fruit (persea gratissima gaertner) on calcium utilization in rats" CAPLUS, 1980, XP002976922
- ESLAVA C A ET AL: "Aqueous extracts of medicinal plants, interfere with grow and adherence to HEp-2 culture cells of bacterial strains, associated with diarrhea disease" ABSTRACTS OF THE GENERAL MEETING OF THE AMERICAN SOCIETY FOR MICROBIOLOGY, vol. 101, 2001, page 71, XP009044130 & 101ST GENERAL MEETING OF THE AMERICAN SOCIETY FOR MICROBIOLOGY; ORLANDO, FL, USA; MAY 20-24, 2001 ISSN: 1060-2011
- 1994, HAGERS HANDBUCH DER PHARMAZEUTISCHEN PRAXIS DROGEN P-Z 5. AUFLAGE , SPRINGER VERLAG BERLIN HEIDELBERG , XP002318088 le document en entier
- SHAW P E ET AL: "HIGH PERFORMANCE LIQUID CHROMATOGRAPHIC ANALYSIS OF D MANNO HEPTULOSE PERSEITOL GLUCOSE AND FRUCTOSE IN AVOCADO CULTIVARS" JOURNAL OF AGRICULTURAL AND FOOD CHEMISTRY, vol. 28, no. 2, 1980, pages 379-382, XP009053733 ISSN: 0021-8561

## Description

La présente invention concerne l'utilisation d'une composition comprenant du D-mannoheptulose et/ou du perséitol pour la fabrication d'un médicament destiné au traitement et/ou à la prévention de maladies liées à une modification de l'immunité innée.

Toutes les espèces animales sont confrontées de façon quotidienne à un grand nombre de microorganismes, tels que des bactéries, des champignons, des parasites ou des virus, qui peuvent affecter leur santé voire leur survie. Deux systèmes de défense s'opposent à ces microorganismes : un système appelé immunité innée, qui est commun à tous les animaux, y compris l'homme, et un système immunitaire dit adaptatif ou spécifique qui est acquis grâce aux cellules et aux médiateurs de l'immunité après contact avec l'agresseur potentiel.

Une différence entre les réponses immunitaires innées ou adaptatives se situe au niveau des mécanismes de reconnaissance des microorganismes impliqués. Dans l'immunité innée, la spécificité des récepteurs est génétiquement déterminée dès la naissance et elle n'est pas variable. Ces récepteurs sont exprimés sur des cellules telles que certaines cellules épithéliales et endothéliales, les cellules dendritiques, les monocytes et les macrophages. Toutes les structures reconnues par les récepteurs de l'immunité innée sont communes à de très nombreux microorganismes. Contrairement à la réponse immunitaire adaptative, les mécanismes de la réponse immunitaire innée (phagocytose, peptides antimicrobiens, ...) sont activés dès le début de l'infection et contrôlent de façon quasi-immédiate la prolifération des pathogènes qui envahissent l'hôte. Ensuite, la réponse immunitaire adaptative prend le relais.

Les peptides anti-microbiens ont été retrouvés à la fois dans le règne végétal et animal, et plus de 500 peptides anti-microbiens différents ont été découverts des insectes à l'homme. Les peptides anti-microbiens sont des molécules de petite taille (10 à 50 acides aminés) capables de détruire une grande variété de micro-organismes (bactéries Gram+ et/ou Gram-, champignons, virus, cellules transformées), en perméabilisant leur membrane cellulaire. En outre, certains de ces peptides anti-microbiens via des propriétés chemo-attractives sont capables de recruter des cellules participant à l'immunité adaptative telles que les cellules dendritiques ou bien encore les lymphocytes T. De nombreux peptides anti-microbiens ont été mis en évidence dans le vernix caseosa et dans le fluide amniotique, ainsi que dans la peau des nouveaux nés, suggérant leur rôle clé dans la défense anti-microbienne lors de l'accouchement, mais également dès les début de la vie alors que l'immunité acquise est encore immature.

La plupart des organismes synthétisent plusieurs types de peptides anti-microbiens au niveau de leurs différents épithélia, de manière à définir un large spectre d'activité. Chez les mammifères, deux grandes classes de peptides anti-microbiens, dont la production est induite lors d'un contact avec un micro-organisme, ont été décrites : les cathélicidines et les défensines.

La cathélicidine humaine (LL-37) a été isolée pour la première fois à partir de cellules de la moelle osseuse. LL-37 est exprimée notamment dans la peau humaine, au niveau des ongles, ainsi qu'au niveau de la membrane synoviale saine et inflammatoire, notamment chez des patients atteints d'arthrose. LL-37 possède un large spectre d'activité, et semble agir en synergie avec d'autres peptides antimicrobiens notamment les défensines. LL-37 possèdent également des propriétés chémo-attractantes, ce qui lui confère la capacité de pouvoir recruter des cellules de l'immunité adaptative.

Les défensines sont elles-mêmes divisées en deux familles, α et β, sur la base de leur structure secondaire. Les α-défensines (6 connues à ce jour) sont principalement localisées dans les granules de stockage des cellules spécialisées telles que les neutrophiles, ou les cellules de Paneth intestinales, alors que les β-défensines sont caractéristiques des tissus épithéliaux. Outre leur rôle dans l'immunité innée, les défensines sont également connues pour leurs propriétés mitogéniques, ce qui suggère leur implication potentielle dans les processus de cicatrisation.

Chez l'homme, 4 β-défensines ont été identifiées à ce jour (il existerait plus de 20 gènes codant pour des peptides anti-microbiens dans notre génome). La β-défensine 1 (hBD-1) humaine est généralement produite de manière constitutive et est exprimée en quantité importante dans les reins et dans une moindre proportion au niveau du pancréas, des glandes salivaires, des épithélia des voies respiratoires, dans le système uro-génital de la femme, dans la membrane synoviale saine, ainsi que dans le placenta. hBD-1 est également exprimée dans la peau. Les autres formes de β-défensines, hBD-2, 3 et 4, sont inductibles. hBD-3 est induite au niveau des membranes synoviales inflammatoires comme par exemple dans les pathologies arthrosiques. L'expression de hBD-2 a été rapportée, à ce jour, dans la peau, le tractus uro-génital, les glandes sudorales, et au niveau de l'unité pilo-sébacée.

Au niveau de la peau, d'autres peptides ou protéines, tels que l'adrénomedulline, la cystatine, l'inhibiteur spécifique de l'élastase/SKALP/elafin, posséderaient des activités anti-microbiennes. Plus récemment, la dermicidine (large spectre d'activité) a été caractérisée comme un peptide anti-microbien spécifique de la peau qui serait produit au niveau des glandes sudorales eccrines, et dont la sécrétion simultanée avec la sueur constituerait une part importante du système de défense inné contre les infections locales et systémiques. hBD-2 a été caractérisée pour la première fois dans les squames de psoriasis. L'expression de hBD-2 et -3, ainsi que de LL-37, est augmentée au niveau des lésions de psoriasis, ce qui expliquerait la plus grande résistance aux infections des patients atteints de cette pathologie. A l'inverse, dans la dermatite atopique (lésions chroniques et lésions en poussée), l'expression de LL-37 et de hBD-2 est diminuée sous l'influence de l'interleukine-4 (IL-4) et de l'interleukine-13 (IL-13), médiateurs de l'atopie. Cette déficience pourrait expliquer la susceptibilité accrue aux infections des patients atteints de dermatite atopique. Dans l'acné, l'expression des β-défensines (hBD-1 et -2) est augmentée en réaction à la prolifération de *P acnes.* En outre, il est supposé que les acnéiques souffriraient d'un déséquilibre initial en peptides anti-microbiens, responsable de la prolifération bactérienne, bactéries qui en retour stimuleraient les défenses immunitaires innées.

L'inflammation semble donc être un facteur prépondérant dans l'induction des peptides anti-microbiens. Ainsi, il a été également monté que l'interleukine-1, le TNF-α (Tumor Necrosis alpha), et l'irradiation ultra-violette stimulaient la production de hBD-2. L'expression de hBD-2 est également liée à l'état de différenciation des kératinocytes. Ainsi, la stimulation de la production des peptides anti-microbiens, notamment de la famille des défensines, et plus particulièrement de hBD-2, permettrait de promouvoir et/ou de rétablir l'immunité innée, notamment au niveau des yeux et des épithélia (épiderme, muqueuses vaginale, intestinale, nasale et auriculaires, voies respiratoires).

La cavité buccale est constamment exposée à une grande variété de microbes (bactéries, virus, champignons). Entre autre, il est bien établi que des bactéries telles que actinobacillus actinomycetemcomitans, porphyromonas gingivalis sont des facteurs clé participant au développement des maladies parodontales (gingivite et parodontite). L'épithélium gingival constitue le premier rempart contre les différents pathogènes présents dans la sphère buccale. A ce titre, les kératinocytes gingivaux produisent un large panel de peptides anti-microbiens, hBD-1, -2, -3, LL37. Ces peptides sont également produits au niveau de la muqueuse buccale, ainsi que par les glandes salivaires.

Plus particulièrement, cette stimulation permettrait de promouvoir et/ou de rétablir l'immunité innée au niveau de la peau saine ou pathologique des nourrissons et des enfants, dont l'immunité est généralement déficiente, et au niveau de la peau des adultes ou des personnes âgées en bonne santé ou malades (immuno-déprimés). Cette stimulation permettrait ainsi de compléter avantageusement le système de défense passif de la peau que constitue le stratum corneum (cornéocytes + ciment intercellulaire), et de préparer la réponse immunitaire adaptative chez les nourrissons, les enfants, les adultes et les personnes âgées, en bonne santé ou malades. Parallèlement, cette stimulation permettrait de promouvoir la cicatrisation.

Le document FR 2 843 125 décrit des principes actifs capables de stimuler l'expression des hBD-2 et h-BD-3. Il peut s'agir de la racine d'armoise, la vergerette du Canada, l'écorce de sureau, l'herniaire, le jus d'ananas, la menthe poivrée, l'Aréquier, le cacaoyer, le quinoa, l'arnica, le boldo, la salsepareille, la feuille de noyer, la fleur d'hibiscus, le potiron, le tournesol, la pivoine, le millepertuis, le marronnier d'inde ou l'un de leurs extraits ; l'acide jasmonique ou la vitamine A, leurs dérivés et leurs précurseurs ; l'alpha-MSH ou un des peptides constituant l'alpha-MSH ou une structure chimique mimant un de ces peptides ; les esters de l'isoleucine ; le calcium ou tous les sels organiques ou minéraux de calcium.

Le D-mannoheptulose, premier cétohéptose identifié en 1916 par La Forge, de formule générale (I) 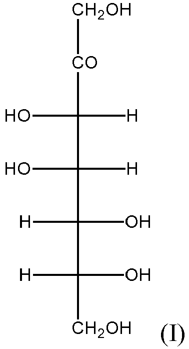 se retrouve dans certaines plantes, en particulier dans la luzerne (Medicago sativa L.), dans l'avocat, dans la figue (Ficus officinalis L.) et dans la primevère (Primula officinalis Jacq.). Toutefois, c'est dans l'avocat, que l'on retrouve les teneurs les plus importantes en D-mannoheptulose. Le D-mannoheptulose a déjà été utilisé dans des applications thérapeutiques. Par exemples, la demande de brevet WO95/03809 décrit l'utilisation du D-mannoheptulose, en tant qu'inhibiteur de glucokinase, pour inhiber le développement des cellules tumorales et la demande US2003/0092669 décrit un complément alimentaire oral comprenant du D-mannoheptulose, qui permet de diminuer le taux d'insuline et qui permet ainsi une perte de poids.

Le perséitol, forme polyol du D-mannoheptulose, de formule générale (II) 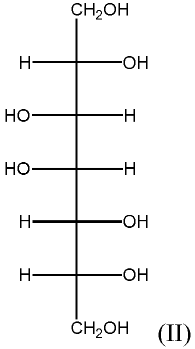 se retrouve également dans l'avocat, en particulier dans le fruit ou dans le noyau de l'avocat.

D'après la publication « Search for pharmacochemical leads from tropical rainforest plants », Hitotaka Shibuya et al. Pure Appl. Chem., vol. 71, n°6, pp 1109-1113, 1999, le perséitol, associé à un ion potassium, permet d'inhiber l'incorporation de leucine-³H dans des cellules tumorales d'ascite sarcomateuse d'Ehrlich.

D'une manière surprenante, les inventeurs ont découvert qu'une composition comprenant du D-mannoheptulose et/ou du perséitol permet l'augmentation de la production de peptides antimicrobiens, avantageusement de la hBD-2, sans induire de réactions inflammatoires, d'irritations ou d'intolérances notamment sans stimuler de manière significative la sécrétion de molécules habituellement exprimées dans le cas de réactions inflammatoires.

Ainsi, la présente invention a pour objet l'utilisation d'une composition comprenant du D-mannoheptulose et/ou du perséitol et un excipient approprié pharmaceutiquement acceptable, pour la fabrication d'un médicament ou d'une composition vétérinaire destiné au traitement et/ou à la prévention de maladies liées à une modification de l'immunité innée **et/ou acquise** par augmentation de la production de peptides antimicrobiens, de la famille des bétâ-défensines, avantageusement de la hBD-2. Au sens de la présente invention, le terme « modification » peut signifier augmentation ou diminution.

Il est décrit également l'utilisation d'une composition comprenant du D-mannoheptulose et/ou du perséitol et un excipient approprié pharmaceutiquement acceptable, pour la fabrication d'un médicament ou d'une composition vétérinaire destiné au traitement et/ou à la prévention de maladies liées à une modification de l'immunité innée **et/ou acquise** par stimulation des peptides anti-microbiens like, tels qu'un inhibiteur spécifique de l'élastase, particulièrement l'élafin (SKALP).

### Le médicament ou la composition vétérinaire selon l'invention permet avantageusement de stimuler et/ou de compléter l'immunité innée et/ou l'immunité acquise.

D'une manière générale, dans le cadre de la présente invention, lesdites maladies peuvent être liées à la présence de micro-organismes, notamment de bactéries Gram+ et/ou Gram-, de champignons, de levures ou de virus.

Plus particulièrement, lesdites maladies peuvent être des infections des systèmes oculaire et auditif, des épithélia non kératinisés (muqueuse vaginale, intestinale, gingivale, nasale, pulmonaire, tractus respiratoire, anale et uréthrale, pulmonaire) et kératinisés tels que la peau. Les dites maladies peuvent également être des infections des phanères ou annexes cutanées (cheveux, ongles, glandes sudorales, glandes sébacées). Ainsi lesdites maladies peuvent être des pathologies telles que la folliculite, le furoncle, l'abcès, l'impétigo ou le panaris.

Lesdites maladies peuvent être des pathologies du cuir chevelu telles que les pellicules, et plus largement des affections liées à une hyper-séborrhée.

Lesdites maladies peuvent être des pathologies liées à une modification de la balance Th1/Th2 telles que la dermatite atopique.

Lesdites maladies peuvent être des pathologies associées à une modification de la synthèse de cytokines, telles que l'IL-4 et/ou l'IL-13, notamment dans le cadre de la dermatite atopique.

Lesdites maladies peuvent également être des dermatoses inflammatoires, telles que la dermatite atopique, l'eczéma atopique et/ou de contact, le psoriasis, l'acné, et des dermatites irritatives.

Lesdites maladies peuvent aussi être des brûlures, en particulier des brûlures du premier ou second degré.

Lesdites maladies peuvent également être des pathologies liées à un déficit de la barrière cutanée. Ainsi, le médicament selon l'invention peut être utilisé pour le traitement des peaux hyper-réactives (sensibles, irritées, allergiques), atopiques, sèches ou âgées. Lesdites maladies peuvent aussi être des pathologies liées à des peaux fragilisées par une agression environnementale, notamment due au froid, à la pollution, au stress, au tabac, à des expositions solaires.

Dans le cadre de la présente invention, le médicament est également approprié pour la protection des peaux immatures, saines ou pathologiques, des nourrissons et des enfants. En effet, il permet de renforcer les défenses naturelles de l'épiderme de l'enfant dont l'immunité est généralement déficiente.

Dans le cadre de la présente invention, le médicament est également approprié pour la protection des peaux saines ou pathologiques des adultes ou des personnes âgées, notamment des individus immuno-déprimés.

Le médicament selon l'invention est également approprié pour favoriser la cicatrisation, dans les processus de cicatrisation normaux ou pathologiques, tels que les ulcères et les escarres.

Dans le cadre de la présente invention, le médicament est aussi destiné à traiter et/ou prévenir les maladies parodontales, les pathologies articulaires inflammatoires telle que l'arthrose, les infections des muqueuses, notamment des muqueuses vaginale, intestinale, respiratoire, nasale ou auriculaire, ou les infections du système oculaire.

La composition selon l'invention comprend avantageusement 0,001 à 30 % en poids sec de D-mannoheptulose, par rapport au poids total de ladite composition, encore plus avantageusement 0,01-20% en poids sec, encore plus avantageusement 0,1-10% en poids sec, encore plus avantageusement 0,5-5% en poids sec de D-mannoheptulose. La composition selon l'invention comprend avantageusement 0,001 à 30 % en poids sec de perséitol, par rapport au poids total de ladite composition, encore plus avantageusement 0,01-20% en poids sec, encore plus avantageusement 0,1-10% en poids sec, encore plus avantageusement 0,5-5% en poids sec de perséitol.

La source de D-mannoheptulose et/ou de perséitol peut être un extrait hydrosoluble de sucres d'avocat ou d'une autre plante. Autrement, le D-mannoheptulose et le perséitol sont disponibles commercialement (origine synthétique). Selon une variante avantageuse de l'invention, la source de D-mannoheptulose et/ou de perséitol est un extrait hydrosoluble de sucres d'avocat.

L'extrait hydrosoluble de sucres d'avocat peut directement être obtenu à partir de n'importe quelle partie de l'avocat ou de l'avocatier, telle que le fruit, la peau, le noyau, la feuille ou les racines de l'avocatier. Il est aussi possible d'obtenir un extrait peptidique d'avocat à partir des co-produits de l'industrie de transformation de l'avocat, parmi lesquels on peut citer de façon non exhaustive : la pulpe fraîche d'avocat, la pulpe congelée, déshydratée, les tourteaux d'avocat issus des procédés d'extraction d'huile (extraction mécanique et/ou par solvant du fruit préalablement déshydraté), les matières solides déshuilées issues des procédés d'extraction d'huile par voie humide (procédé dit de centrifugation), les matières solides déshuilées issues des procédés d'extraction d'huile d'avocat par voie enzymatique, les purées d'avocat brutes (guacamole), les déchets solides issus des unités de production de ces purées. L'extrait est avantageusement obtenu à partir du fruit frais de l'avocatier. Les fruits pourront être choisis parmi les variétés *Hass, Fuerte, Ettinger, Bacon, Nabal, Anaheim, Lula, Reed, Zutano, Queen, Criola Selva, Mexicana Canta, Region Dschang, Hall, Booth, Peterson, Collinson Redn,* plus avantageusement parmi les variétés *Hass, Fuerte et Reed.* De préférence, on retiendra les variétés *Hass, Fuerte, Ettinger et Bacon,* et plus avantageusement les variétés *Hass* et *Fuerte.*

Le fruit de l'avocatier est principalement constitué d'eau, de pulpe, d'huile et de noyau. Les proportions de ces constituants sont, à l'instar de toutes matières naturelles et végétales, extrêmement variables. Toutefois, on admet généralement les données de composition moyennes suivantes, exprimées en pourcentage de fruit frais, données dans le tableau 1 suivant :

**Tableau 1**

| | |
|---|---|
| Eau | 70-85 % |
| Protéines | 1,5-4,5 % |
| Lipides | 12-23 % |
| Sucres | 1,5-5 % |
| Fibres | 1,1-1,6 % |

De fait, l'avocat n'est pas particulièrement riche en polysaccharides. Cependant, la nature des monosaccharides solubles est tout à fait particulière, tels que le perséitol ou le D-mannoheptulose constitués de 7 atomes de carbone.

L'extrait hydrosoluble de sucres d'avocat est susceptible d'être obtenu par un procédé comprenant les étapes successives suivantes :
- obtention d'un tourteau d'avocat, avantageusement du fruit de l'avocat, par séchage de l'avocat puis extraction des lipides (huile) ; ensuite
- cryobroyage et délipidation complète dudit tourteau, puis décantation et centrifugation afin de récupérer une fraction soluble riche en sucres en C7 (élimination du gâteau) ;
- déminéralisation sur résine ionique de ladite fraction soluble, obtenue à l'étape précédente ; puis
- ultrafiltration à 10 000 daltons ; et
- le cas échéant, concentration sous vide et conditionnement.

La première étape du procédé consiste à sécher le fruit puis à le déshuiler. Ainsi, après tranchage du fruit en fines lamelles, son séchage peut être réalisé par l'ensemble des techniques connues de l'homme de métier, parmi lesquelles on peut citer le séchage à l'air chaud, la lyophilisation ou encore le séchage osmotique. D'une façon générale, la température lors de cette étape de séchage sera avantageusement maintenue, quelle que soit la technique employée, inférieure ou égale à 80°C. Dans le cadre du présent procédé, pour des raisons de facilité de mise en œuvre et pour des raisons de coût, le séchage en séchoirs ventilés, en couche mince et sous courant d'air chaud, à une température comprise entre 70 et 75°C est préféré. La durée de l'opération peut varier de 5 à 72 heures.

Les lipides du fruit séché sont par la suite extraits soit par voie mécanique dans une presse à vis continue, ou encore par voie chimique, à l'aide d'un solvant tel que l'hexane, dans un extracteur de type soxhlet ou dans un extracteur continu à bande, de type De Smet®, notamment selon le procédé décrit dans la demande FR 2 843 027, ou par un procédé utilisant le CO₂ supercritique. Parmi les intérêts majeurs du procédé, l'huile co-produite constitue un produit bien évidemment directement valorisable. C'est pourquoi, on préfère l'extraction des lipides par voie mécanique. Le fruit sec et déshuilé, ou encore appelé tourteau peut subir ensuite les étapes suivantes :
- cryobroyage
- délipidation complète, notamment à l'acétone et/ou à l'éthanol,
- décantation puis lavage du tourteau à l'eau,
- centrifugation, récupération de la fraction soluble (élimination du gâteau),
- déminéralisation par échange d'ions
- ultrafiltration avec un seuil de coupure de 10 kD
- concentration sous vide, ajout de conservateur et conditionnement.

De façon générale, l'extrait aqueux final peut contenir en poids 0,1 à 10 % de matière sèche, avantageusement 1 à 7 % de matière sèche, encore plus avantageusement 3 à 5 % de matière sèche. La teneur en sucres en C7, c'est-à-dire en D-mannoheptulose et en perséitol, dans la matière sèche est avantageusement comprise en 65 à 90% en poids, par rapport au poids total de la matière sèche. Les données analytiques moyennes d'une solution aqueuse à 5 % d'extrait sec, obtenue par le procédé décrit précédemment, sont données dans le tableau 2 suivant :

**Tableau 2**

| | | |
|---|---|---|
| pH (dilution ¼) | | 4,5 - 7,5 |
| Absorbance (dilution ½) | 420 nm | Inférieure à 0,2 |
| | 550 nm | Inférieure à 0,05 |
| Sucres en C7 / matière sèche | | 65 - 90 % |

La composition relative en sucres de l'extrait hydrosoluble, en poids par rapport au poids total de la matière sèche de l'extrait, répond avantageusement aux critères suivants (composition relative déterminée par HPLC ; high performance liquid chromatography = chromatographie liquide haute performance):

| | |
|---|---|
| - D-mannoheptulose | 5 à 80 % |
| - Perséitol | 5 à 80 % |
| - Saccharose | inférieur à 10% |
| - Glucose | inférieur à 10% |
| - Fructose | inférieur à 10% |

L'extrait hydrosoluble de sucre d'avocat comprend avantageusement, par rapport au poids total de la matière sèche, 10 à 80% en poids de D-mannoheptulose plus avantageusement 15 à 70 % en poids de D-mannoheptulose. L'extrait hydrosoluble de sucre d'avocat comprend avantageusement, par rapport au poids total de la matière sèche, 20 à 80% en poids de perséitol, plus avantageusement 25 à 70% en poids de perséitol.

De préférence, la composition relative en sucres de l'extrait hydrosoluble, en poids par rapport au poids total de la matière sèche de l'extrait, répond aux critères suivants (composition relative déterminée par HPLC) :

| | |
|---|---|
| - D-mannoheptulose | 25 à 60 % |
| - Perséitol | 25 à 60 % |
| - Saccharose | inférieur à 10 % |
| - Glucose | inférieur à 10 % |
| - Fructose | inférieur à 10 % |

D'une manière surprenante les inventeurs ont constaté un effet de synergie entre le D-mannoheptulose et/ou le perséitol et les sucres minoritaires (fructose, glucose, saccharose) présents dans l'extrait de sucres d'avocat.

L'extrait obtenu pourra éventuellement être lyophilisé dans le but d'obtenir une poudre solide (extrait sec), totalement hydrosoluble.

Selon une variante avantageuse de l'invention, la composition comprend en outre un extrait peptidique d'avocat, avantageusement en une proportion de 0,001 à 30% en poids sec, encore plus avantageusement 0,01 à 20% en poids sec, encore plus avantageusement 0,1 à 15% en poids sec, encore plus avantageusement 0,5 à 10% en poids sec, encore plus avantageusement 0,7 à 8% en poids sec, et encore plus avantageusement 1 à 5% en poids sec, par rapport au poids total de la composition. On observe alors avantageusement un effet de synergie.

L'extrait peptidique d'avocat, ajouté dans la composition selon l'invention, comprend avantageusement 2 à 10% en poids d'azote alpha-aminé, par rapport au poids de la matière sèche de l'extrait peptidique. Dans le cadre de la présente invention, on entend par les termes « azote alpha-aminé », la teneur en azote des peptides sous la forme de groupes alpha-aminés libres. La mesure de la teneur en azote alpha-aminé des peptides permet d'évaluer le degré d'hydrolyse des protéines ainsi que la masse molaire moyenne des peptides.

Plus particulièrement, l'extrait peptidique d'avocat est susceptible d'être obtenu par un procédé comprenant les étapes suivantes :
- obtention d'un tourteau d'avocat, avantageusement du fruit de l'avocat, par séchage de l'avocat puis extraction des lipides ; ensuite
- cryobroyage et délipidation complète dudit tourteau puis décantation, centrifugation et récupération du gâteau ; puis
- première hydrolyse en présence de glucanases, suivie d'une centrifugation et de l'élimination de la fraction soluble ;
- seconde hydrolyse en présence d'une ou plusieurs protéases, suivie d'une centrifugation et de l'élimination du culot ; ensuite
- concentration de la phase peptidique par nanofiltration ;
- décoloration, en présence de charbon actif par exemple, suivie d'une filtration simple (10 µm) puis d'une ultrafiltration (seuil de coupure de 10 kD) ; enfin
- le cas échéant, micro filtration stérilisante finale (0,2 µm), ajout de conservateur et conditionnement.

L'obtention du tourteau d'avocat et l'extraction des lipides sont avantageusement effectuées d'une manière identique pour l'extrait peptidique d'avocat et les sucres d'avocat. Le fruit sec et déshuilé, ou encore appelé tourteau peut subir ensuite les étapes suivantes :
- cryobroyage
- délipidation complète, notamment à l'éthanol et/ou à l'acétone,
- décantation, puis lavage du tourteau à l'eau,
- centrifugation, récupération du gâteau,
- première hydrolyse en présence d'une ou plusieurs glucanases,
- centrifugation, élimination de la fraction soluble,
- seconde hydrolyse en présence d'une ou plusieurs protéases,
- centrifugation, élimination du culot,
- concentration par nanofiltration
- décoloration, notamment en présence de charbon actif,
- filtration simple (10 µm) puis ultrafiltration (seuil de coupure de 10 kD),
- conditionnement et ajout de conservateur,
- microfiltration stérilisante finale (0,2 µm)
- ajout de conservateur et conditionnement.

L'extrait aqueux final peut contenir en poids 1 à 60 % de matière sèche, ou encore 3 à 20 % de matière sèche, de préférence 5 à 6 % de matière sèche. L'extrait obtenu pourra éventuellement être lyophilisé dans le but d'obtenir une poudre solide (extrait sec), mais totalement hydrosoluble par rapport aux protéines originelles de l'avocat. Par rapport au poids de la matière sèche, la teneur en azote alpha aminé pourra être comprise entre 2 et 10 % en poids, de préférence entre 5 et 7 % en poids. Le pH d'une solution aqueuse à 1,2 % en poids d'extrait sec, par rapport au poids de la matière sèche, sera généralement compris entre 3 et 6, plus avantageusement entre 4 et 5.

Selon une variante avantageuse de l'invention, la composition comprend en outre un extrait peptidique de lupin, avantageusement en une quantité massique, par rapport au poids total de la composition, de 0,001 à 30% en poids sec, encore plus avantageusement de 0,01 à 10 % en poids sec. L'extrait peptidique de lupin, ajouté dans la composition selon l'invention, comprend au moins 70%, avantageusement au moins 80%, en poids de peptides, par rapport au poids de la matière sèche de l'extrait peptidique. On observe alors avantageusement un effet de synergie.

En particulier, l'extrait peptidique de lupin est susceptible d'être obtenu par un procédé comprenant les étapes suivantes :
- préparation d'un tourteau de lupin broyé ou d'une farine de lupin micronisée ;
- puis, délipidation par extraction avec un solvant
- extraction des fractions protéiques et osidiques solubles, ou précipitation des protéines au point isoélectrique ;
- éventuellement séparation de la fraction protéique ;
- hydrolyse enzymatique de la fraction protéique, et récupération, éventuellement après filtration, de l'extrait peptidique.

Le procédé de préparation d'un extrait peptidique est décrit dans la demande de brevet FR 2 792 202, déposée par les laboratoires Expanscience.

La composition peut en outre comprendre au moins un composé choisi dans le groupe constitué par les émollients, les actifs hydratants, les activateurs de la synthèse de kératine, les kératorégulateurs, les kératolytiques, les agents restructurant de la barrière cutanée (activateurs de la synthèse des lipides cutanés, agonistes PPARs ou Peroxysome Proliferator Activated Receptor), les activateurs de la différenciation des kératinocytes (rétinoïdes, calcidone®, le calcium), les antibiotiques, les agents anti-bactériens, les composés antifongiques, les agents anti-viraux, les sébo-régulateurs, tels que les inhibiteurs de 5-alpha réductase, notamment l'actif 5-alpha Avocuta® commercialisé par les Laboratoires Expanscience, les immunomodulateurs, tels que le tacrolimus, le pimécrolimus, les oxazolines, les conservateurs, les agents anti-irritants, les agents apaisants, des filtres et écrans solaires, les agents anti-oxydants, les facteurs de croissance, les agents cicatrisants ou les molécules eutrophiques, les médicaments et les agents anti-inflammatoires, et les composés contenant des insaponifiables d'huiles végétales.

Les activateurs de la synthèse de kératine pouvant être utilisés dans le cadre de la présente invention en association avec le D-mannoheptulose et/ou perséitol sont avantageusement les rétinoïdes, les peptides de lupin (commercialisés par la société Silab), des protéines clés du stratum corneum ou granulosum (kératines).

Les antibiotiques pouvant être utilisés dans le cadre de la présente invention en association avec le D-mannoheptulose et/ou perséitol sont avantageusement l'acide fucidique, la pénicilline, les tétracyclines, la pristinamycine, l'érythromycine, la clindamycine, la mupirocine, la minocycline, la doxycycline. Les agents anti-viraux pouvant être utilisés dans le cadre de la présente invention en association avec le D-mannoheptulose et/ou perséitol sont avantageusement l'acyclovir et le valacyclovir. Les agents anti-irritants pouvant être utilisés dans le cadre de la présente invention en association avec le D-mannoheptulose et/ou perséitol sont avantageusement la glycine, les sucres et/ou peptides de lupin, le Cyclocéramide® (dérivé d'oxazoline).

Les agents apaisants pouvant être utilisés dans le cadre de la présente invention en association avec le D-mannoheptulose et/ou perséitol sont avantageusement l'alpha bisabolol, les dérivés de réglisse. Les kératorégulateurs pouvant être utilisés dans le cadre de la présente invention en association avec le D-mannoheptulose et/ou perséitol sont avantageusement les alpha hydroxy acides et leurs dérivés. Un kératolytique pouvant être utilisé dans le cadre de la présente invention en association avec le D-mannoheptulose et/ou perséitol est notamment l'acide salicylique et ses dérivés. Les agents anti-oxydants pouvant être utilisés dans le cadre de la présente invention en association avec le D-mannoheptulose et/ou perséitol sont avantageusement les vitamines (C, E), les oligo-éléments (cuivre, zinc, sélénium). Les facteurs de croissance pouvant être utilisés dans le cadre de la présente invention en association avec le D-mannoheptulose et/ou perséitol sont avantageusement la becaplermine et le TGF-beta (Transforming Growth Factor beta).

Les agents cicatrisants pouvant être utilisés dans le cadre de la présente invention en association avec le D-mannoheptulose et/ou perséitol sont avantageusement la vitamine A, le panthénol, l'Avocadofurane®, l'oxyde zinc, le magnésium, le silicium, l'acide madécassique ou asiatique.

Les médicaments pouvant être utilisés dans le cadre de la présente invention, en association avec le D-mannoheptulose et/ou perséitol, sont avantageusement les médicaments, appropriés pour une administration pour voie topique ou orale, pour la prévention et/ou le traitement de l'atopie (corticoïdes, émollients), de l'acné (antibiotiques, péroxyde de benzoyle, rétinoïdes, acide azélaïque, vitamine PP, zinc, cyclines), de l'eczéma (immunomodulateurs, émollients, huile de saumon, de bourrache, les pré-biotiques) ou du psoriasis (corticoïdes, calcipotriol, calcitriol, tazarotène, huile de cade, acitrétine, PUVA thérapie). Les agents anti-inflammatoires pouvant être utilisés dans le cadre de la présente invention en association avec le D-mannoheptulose et/ou perséitol sont avantageusement des agents anti-inflammatoires stéroïdiens (AIS), tels que les corticoïdes, ou non-stéroïdiens (AINS).

Les agents restructurant de la barrière cutanée, permettant de stimuler la synthèse des lipides clés de l'épiderme, et pouvant être utilisés dans le cadre de la présente invention en association, avantageusement avec un effet de synergie, avec le D-mannoheptulose et/ou perséitol sont avantageusement des concentrats de tournesol, encore plus avantageusement des concentrats de tournesol linoléiques, tels que l'actif commercialisé par les Laboratoires Expanscience, Soline® (cf. la demande internationale WO 01/21150), des insaponifiables d'huile végétale, tel que l'Avocadofurane® (cf. la demande internationale WO 01/21150), des agonistes PPARs (rosiglitazone, pioglitazone). Les agents restructurant sont avantageusement présent en une proportion allant de 0,001 à 30 % en poids, par rapport au poids total de la composition ou du médicament. Les composés antifongiques pouvant être utilisés dans le cadre de la présente invention en association avec le D-mannoheptulose et/ou perséitol sont avantageusement l'econazole et le ketoconazole.

Les conservateurs antiseptiques pouvant être utilisés dans le cadre de la présente invention en association avec le D-mannoheptulose et/ou perséitol sont par exemple le triclosan, la chlorhéxidine, les ammoniums quaternaires. Les immnumodulateurs pouvant être utilisés dans le cadre de la présente invention en association, avantageusement avec un effet de synergie, avec le D-mannoheptulose et/ou perséitol sont avantageusement le tacrolimus, le pimécrolimus et les oxazolines.

Les oxazolines pouvant être utilisées dans le cadre de la présente invention en association, avantageusement avec un effet de synergie, avec le D-mannoheptulose et/ou perséitol sont avantageusement des oxazolines choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. De manière encore plus avantageuse, ladite oxazoline est la 2-undécyl-4,4-diméthyl-1,3-oxazoline, appelée OX-100 ou Cyclocéramide®.

Les composés contenant des insaponifiables d'huiles végétales pouvant être utilisés dans le cadre de la présente invention en association, avantageusement avec un effet de synergie, avec le D-mannoheptulose et/ou perséitol sont avantageusement choisis dans le groupe constitué par les lipides furaniques d'avocat, les insaponifiables d'avocat et de soja, les concentrats d'huile de lupin, les concentrats d'huile de tournesol et leurs mélanges.

Les lipides furaniques d'avocat pouvant être utilisés dans le cadre de la présente invention en association, avantageusement avec un effet de synergie, avec le D-mannoheptulose et/ou perséitol sont avantageusement des 2-alkyl furanes naturels, notamment l'actif Avocadofurane® commercialisé par les Laboratoires Expanscience, pouvant être obtenus par le procédé décrit dans la demande internationale WO 01/21605.

Les insaponifiables d'avocat et de soja pouvant être utilisés dans le cadre de la présente invention en association, avantageusement avec un effet de synergie, avec le D-mannoheptulose et/ou perséitol sont avantageusement un mélange d'insaponifiables d'avocat furanique et d'insaponifiables de soja, dans un rapport respectif d'environ 1/3-2/3. Les insaponifiables d'avocat et de soja sont encore plus avantageusement le produit Piasclédine®, commercialisé par les Laboratoires Expanscience.

Les concentrats d'huile de lupin pouvant être utilisés dans le cadre de la présente invention en association, avantageusement avec un effet de synergie, avec le D-mannoheptulose et/ou perséitol sont avantageusement des concentrats obtenus par distillation moléculaire d'huile de lupin, avantageusement d'huile de lupin blanc doux, tels que ceux décrits dans la demande internationale WO 98/47479. Ils contiennent avantageusement environ 60% en poids d'insaponifiables.

Les concentrats d'huile de tournesol pouvant être utilisés dans le cadre de la présente invention en association, avantageusement avec un effet de synergie, avec le D-mannoheptulose et/ou perséitol sont avantageusement des concentrats de tournesol linoléiques, tels que l'actif commercialisé par les Laboratoires Expanscience, Soline® (cf. la demande internationale WO 01/21150).

La composition selon l'invention peut être formulée sous la forme de différentes préparations adaptées à une administration topique, à une administration orale, rectale, vaginale, nasale, auriculaire ou bronchique, à une administration parentérale. De préférence les différentes préparations sont adaptées à l'administration topique et incluent les crèmes, les pommades, les lotions, les huiles, les patchs, les sprays ou tout autres produits pour application externe. Les modes d'administration, les posologies et les formes galéniques optimales des composés et compositions selon l'invention peuvent être déterminés selon les critères généralement pris en compte dans l'établissement d'un traitement pharmaceutique, en particulier dermatologique, ou vétérinaire adapté à un patient ou à un animal comme par exemple l'âge ou le poids corporel du patient ou de l'animal, la gravité de son état général, la tolérance au traitement, les effets secondaires constatés, le type de peau. En fonction du type d'administration souhaitée, la composition et/ou les composés actifs selon l'invention peuvent en outre comprendre au moins un excipient pharmaceutiquement acceptable, notamment dermatologiquement acceptable. De préférence, on utilise un excipient adapté pour une administration par voie topique externe. La composition selon la présente invention peut en outre comprendre au moins un adjuvant pharmaceutiquement connu de l'homme du métier, choisi parmi les épaississants, les conservateurs, les parfums, les colorants, des filtres chimiques ou minéraux, les agents hydratants, les eaux thermales, etc.

Le médicament ou la composition vétérinaire selon l'invention peut comprendre 0,001 à 30% en poids de D-mannoheptulose et 0,001 à 30% en poids de perséitol, par rapport au poids total dudit médicament, encore plus avantageusement 0,01 à 10% en poids de D-mannoheptulose et 0,01 à 10% en poids de perséitol, et un excipient approprié pharmaceutiquement acceptable.

Le médicament ou la composition vétérinaire selon l'invention peut être formulé sous la forme de différentes préparations adaptées à une administration topique, à une administration orale ou rectale, vaginale, urétrale, auriculaire, bronchique, nasale, à une administration parentérale. Les modes d'administration, les posologies et les formes galéniques optimales du médicament ou de la composition vétérinaire selon l'invention peuvent être déterminés selon les critères généralement pris en compte dans l'établissement d'un traitement pharmaceutique, en particulier dermatologique, adapté à un patient, ou vétérinaire.

La présente invention concerne également une composition cosmétique comprenant 0,001 à 30% en poids de D-mannoheptulose, par rapport au poids total de ladite composition, et 0,001 à 30% en poids de perséitol, par rapport au poids total de ladite composition, encore plus avantageusement 0,01 à 10% en poids de perséitol et 0,01 à 10% en poids de D-mannoheptulose, pour son utilisation dans le traitement des peaux et/ou des muqueuses sensibles, irritées, sèches, âgées, intolérantes, présentant un trouble de la barrière cutanée, fragilisées par une agression environnementale présentant des rougeurs cutanées ou présentant un déséquilibre immunologique non pathologique, par application de la composition sur la peau et/ou les muqueuses.
Selon une variante avantageuse de l'invention, la source de D-mannoheptulose et/ou de perséitol est un extrait hydrosoluble de sucres d'avocat, qui peut être obtenu selon un procédé tel que décrit précédemment. Il existe en effet une synergie entre le D-manoheptulose, le perséitol et les sucres minoritaires de l'avocat (fructose, glucose, saccharose).

Selon une variante avantageuse de l'invention, la composition comprend en outre un extrait peptidique d'avocat, avantageusement en une quantité synergétique. L'extrait peptidique d'avocat est avantageusement présent en une quantité de 0,001 à 30 %, plus avantageusement de 0,1 à 10 %, en poids sec, par rapport au poids total de la composition. L'extrait peptidique d'avocat, ajouté dans la composition selon l'invention, comprend avantageusement 2 à 10% en poids d'azote alpha-aminé, par rapport au poids de la matière sèche de l'extrait peptidique. Il peut être obtenu selon un procédé tel que décrit précédemment.

Selon une variante avantageuse de l'invention, la composition comprend en outre un extrait peptidique de lupin, avantageusement en une quantité synergétique. L'extrait peptidique de lupin est avantageusement présent en une quantité de 0,001 à 30 %, plus avantageusement de 0,1 à 10 %, en poids sec, par rapport au poids total de la composition. L'extrait peptidique de lupin, ajouté dans la composition selon l'invention, comprend au moins 70%, avantageusement au moins 80%, en poids de peptides, par rapport au poids de la matière sèche de l'extrait peptidique. Il peut être obtenu selon un procédé tel que décrit précédemment.

La composition peut en outre comprendre au moins un composé choisi dans le groupe constitué par les agents restructurants de la barrière cutanée et les composés contenant des insaponifiables d'huiles végétales, tels que définis précédemment avantageusement en une quantité synergétique. En particulier, la composition cosmétique peut comprendre un actif choisi dans le groupe constitué par Soline®, Avocadofurane® et piasclédine®, commercialisés par les Laboratoires Expanscience. La composition cosmétique selon l'invention comprend avantageusement 0,001 à 30 % en poids, par rapport au poids total de la composition, d'au moins un agent restructurant de la barrière cutanée.

La composition cosmétique selon l'invention peut être formulée sous la forme de différentes préparations adaptées à une administration topique, à une administration orale ou rectale, vaginale, urétrale, auriculaire, nasale, bronchique. De préférence les différentes préparations sont adaptées à l'administration topique et incluent les crèmes, les pommades, les lotions, les huiles, les patches, les sprays ou tout autres produits pour application externe. En fonction du type d'administration souhaitée, la composition et/ou les composés actifs selon l'invention peuvent en outre comprendre au moins un excipient cosmétiquement acceptable. La composition cosmétique selon la présente invention peut en outre comprendre au moins un adjuvant cosmétiquement connu de l'homme du métier, choisi parmi les épaississants, les conservateurs, les parfums, les colorants, des filtres chimiques ou minéraux, les agents hydratants, les eaux thermales, etc.

Il est aussi décrit une méthode de traitement cosmétique des peaux et/ou des muqueuses sensibles, irritées, allergiques, sèches, âgées, intolérantes, présentant un trouble de la barrière cutanée, fragilisées par une agression environnementale, présentant des rougeurs cutanées ou présentant un déséquilibre immunologique non pathologique, caractérisée en ce qu'elle consiste à appliquer sur la peau et/ou les muqueuses une composition cosmétique selon l'invention.

Il est décrit une composition nutraceutique comprenant du D-mannoheptulose et/ou du perséitol et un excipient approprié alimentaire acceptable. La composition nutraceutique comprend avantageusement 0,001 à 30% en poids de D-mannoheptulose, par rapport au poids total de ladite composition, encore plus avantageusement 0,01 à 10% en poids de D-mannoheptulose. La composition nutraceutique comprend avantageusement 0,001 à 30% en poids de perséitol, par rapport au poids total de ladite composition, encore plus avantageusement 0,01 à 10% en poids de perséitol. La composition nutraceutique comprend avantageusement 0,001 à 30% en poids de perséitol et 0,001 à 30% en poids de D-mannoheptulose, par rapport au poids total de ladite composition, encore plus avantageusement 0,01 à 10% en poids de perséitol et 0,01 à 10% en poids de D-mannoheptulose. Selon une variante avantageuse, la source de D-mannoheptulose et/ou de perséitol est un extrait hydrosoluble de sucres d'avocat, qui peut être obtenu selon un procédé tel que décrit précédemment.

La composition peut comprendre en outre un extrait peptidique d'avocat, avantageusement en une quantité synergétique, avantageusement en une quantité de 0,001 à 30 % en poids sec, par rapport au poids total de la composition. L'extrait peptidique d'avocat, ajouté dans la composition comprend avantageusement 2 à 10% en poids d'azote alpha-aminé, par rapport au poids de la matière sèche de l'extrait peptidique. Il peut être obtenu selon un procédé tel que décrit précédemment.

La composition peut comprendre en outre un extrait peptidique de lupin, avantageusement en une quantité synergétique, avantageusement en une quantité de 0,001 à 30 % en poids sec, par rapport au poids total de la composition. L'extrait peptidique de lupin, ajouté dans la composition, comprend au moins 70%, avantageusement au moins 80%, en poids de peptides, par rapport au poids de la matière sèche de l'extrait peptidique. Il peut être obtenu selon un procédé tel que décrit précédemment.

Les exemples suivants permettent d'illustrer la présente invention.

### Exemple 1 : préparation d'un extrait hydrosoluble de sucres d'avocat

50 kg d'avocats frais, de la variété Hass, sont coupés en fines lamelles de 2 à 5 mm d'épaisseur, noyau compris, à l'aide d'un trancheur à disque. L'outil de séchage est une étuve thermo-régulée à courant d'air chaud. Les avocats tranchés sont répartis sur une épaisseur de 4 à 5 cm sur des clayettes étagées. La température de séchage est fixée à 80°C, sa durée est quant à elle de 48 heures. Une fois séchés, les fruits sont soumis à une pression à froid. Cette opération est réalisée sur une petite presse Komet® de laboratoire.

Les 4 kg de fruits délipidés (tourteau) sont alors broyés à froid puis extraits à reflux, en présence de 25 litres d'éthanol. La poudre épuisée en lipides est alors récupérée par filtration sur Büchner et séchée à l'étuve à 50°C, pendant 5 heures.

Le tourteau est alors lavé à l'eau déminéralisée (10 litres) puis séparé par centrifugation. La fraction soluble (liquide) est reprise pour être purifiée et concentrée selon le mode opératoire suivant :
- *Déminéralisation à l'aide de résines échangeuses d'ions* : déminéralisation des heptuloses par passage sur résines OH⁻, puis sur résine H⁺.
- *Ultrafiltration sur 10 000 Da :* l'ultrafiltration est réalisée avec un système équipé de 4 membranes de seuil de coupure 10 kDa.
- *Concentration sous vide :* la concentration de l'extrait purifié est réalisée à l'aide d'un évaporateur sous vide jusqu'à l'obtention d'une matière sèche voisine de 4 %.
- *Conditionnement :* la concentration de l'extrait est ajustée à 5 % de matière sèche et on ajoute du conservateur, puis on filtre stérilement avec une membrane de 0,2 µm de seuil de coupure et on conditionne.

Le tableau 3 suivant donne la composition de l'extrait de sucres d'avocat en C7, à 5 % de matière sèche, préparé suivant le procédé décrit ci-dessus :

**Tableau 3**

| **Aspect** | Solution de couleur jaune pâle |
|---|---|
| **Critères analytiques** | |
| Matière sèche | 5% |
| pH (dilution ¼) | 7,0 |
| Absorbance à 420 nm (dilution ¼) | 0,013 |
| Absorbance à 550 nm (dilution ¼) | 0,003 |

| **Composition (%/matière sèche)** | |
|---|---|
| Saccharose | 3,0 |
| Glucose | 7,5 |
| D-mannoheptulose | 40,0 |
| Fructose | 10,6 |
| Perséitol | 28,8 |

Suivant ce même procédé, on a préparé deux autres extraits, dont la valeur du pH, l'absorbance et la teneur en sucres en C7 sont données dans le tableau 4 suivant. La teneur en sucres en C7 correspond à la somme du perséitol et du D-mannoheptulose analysée par HPLC.

**Tableau 4**

| Lot | | 1 | 2 |
|---|---|---|---|
| Matière sèche | | 5% | 5% |
| pH (dilution ¼) | | 5,9 | 5,4 |
| Absorbance (dilution ¼) | 420 nm | 0,054 | 0,076 |
| | 550 nm | 0,004 | 0,032 |
| Sucres en C7 / matière sèche | | 80,5 | 83,4 |

### Exemple 2 : préparation d'un extrait peptidique d'avocat

50 kg d'avocats frais, de la variété Hass, sont coupés en fines lamelles de 2 à 5 mm d'épaisseur, noyau compris, à l'aide d'un trancheur à disque. L'outil de séchage est une étuve thermo-régulée à courant d'air chaud. Les avocats tranchés sont répartis sur une épaisseur de 4 à 5 cm sur des clayettes étagées. La température de séchage est fixée à 80°C, sa durée est quant à elle de 48 heures. Une fois séchés, les fruits sont soumis à une pression à froid. Cette opération est réalisée sur une petite presse Komet® de laboratoire. Les 4 kg de fruits délipidés (tourteau) sont alors broyés à froid puis extraits à reflux, en présence de 25 litres d'éthanol. La poudre épuisée en lipides est alors récupérée par filtration sur Büchner et séchée à l'étuve à 50°C, pendant 5 heures.
Le tourteau est alors lavé à l'eau déminéralisée (10 litres) puis séparé par centrifugation. La fraction solide est reprise dans une solution aqueuse, acidifiée par HCl (pH fixé à 5), puis mise en présence de 2 % de cellulases par rapport à la matière sèche. La durée d'hydrolyse est fixée à 6 heures.

Le mélange est ensuite centrifugé à 2000 g en présence d'adjuvant (2,5% p/v). Le culot récupéré subit alors une seconde hydrolyse à pH 8,0, en présence de 0,5 % d'Alcalase® (enzyme commercial de la classe des protéases), à une température de 55°C, pendant 2 heures. L'hydrolyse est régulée à pH constant par ajout continu de soude 2M. La protéase est enfin dénaturée par chauffage pendant 10 minutes, à 85°C.

Le mélange obtenu est centrifugé et le surnageant filtré par passage à travers une membrane de 7,5 µm. Il est ensuite ultrafiltré sur des membranes présentant un seuil de coupure de 10 kD.

L'extrait peptidique brut obtenu à 20 % de matière sèche, est décoloré en présence de 1% de charbon actif Norit®, puis filtré à nouveau à travers une membrane de 7,5 µm. L'extrait décoloré est alors micro filtré (0,2 µm), ajusté en titre à hauteur de 5 % de matière sèche, puis complémenté en conservateur (0,4 % p/v de Phenonip®) et enfin conditionné. Les caractéristiques de l'extrait peptidique d'avocat hydrosoluble à 5 % de matière sèche, obtenu par ce procédé sont données dans le tableau 5 suivant :

**Tableau 5**

| **Aspect** | Solution de couleur légèrement orangée |
|---|---|
| **Critères analytiques** | |
| Matière sèche | 5% |
| pH (dilution ¼) | 4,5 |
| Absorbance à 420 nm (dilution ¼) | 0,152 |
| Absorbance à 550 nm (dilution ¼) | 0,035 |

| **Composition de la matière sèche** | |
|---|---|
| Azote alpha-aminé | 6,7 % |
| Protéines | Non détectées |
| Conservateur Phenonip | 0,4 % |

Suivant ce même procédé, on a préparé d'autres extraits, dont les données analytiques sont données dans le tableau 6 suivant.

**Tableau 6**

| Lot | | 1 | 2 | 3 | 4 |
|---|---|---|---|---|---|
| Azote α-aminé (méthode dite « o-phthalaldehyde » ou « ninhydrine ») (en pourcentage massique dans la matière sèche) | | 4,0 | 6,7 | 8,6 | 6,3 |
| Protéines (en pourcentage massique dans la matière sèche) (N^{∗}6,25)¹ | | 10,4 | 22,1 | 24,9 | 15,5 |
| pH (dilution ¼) | | 4,7 | 4,5 | 5,7 | 5,3 |
| Absorbance (dilution ¼) | 420 nm | 0,315 | 0,150 | 0,982 | 0,499 |
| | 550 nm | 0,062 | 0,033 | 0,264 | 0,075 |

¹ N x 6,25 correspond au dosage de l'azote total (N) d'un échantillon multiplié par un coefficient spécifique pour la protéine dosée. Quand on ne connaît pas précisément le coefficient des protéines dosées, on utilise par convention le coefficient 6,25.

Dans l'aminogramme donné dans le tableau 7 suivant, les valeurs sont exprimées en pourcentage en poids par rapport au poids total des acides aminés dosés.

Les valeurs pour l'acide aspartique et l'acide glutamique incluent également les teneurs en asparagine et glutamine, respectivement.

**Tableau 7**

| Acide aminé | Résultats (moyenne) |
|---|---|
| Alanine | 7,1 |
| Arginine | 5,2 |
| Acide aspartique | 11,5 |
| Cystine | 3,2 |
| Acide glutamique | 14,5 |
| Glycine | 5,9 |
| Histidine | 2,4 |
| Isoleucine | 5,3 |
| Leucine | 8,5 |
| Lysine | 3,4 |
| Méthionine | 1,4 |
| Phénylalanine | 5,2 |
| Proline | 4,7 |
| Sérine | 6,1 |
| Thréonine | 5,1 |
| Tyrosine | 4,0 |
| Valine | 6,5 |
| TOTAL | 100,0 |

| | |
|---|---|
| Tryptophane non dosé | |

Dans les exemples 3 à 7, sauf indication contraire, les pourcentages sont exprimés en poids par rapport au poids total de la matière sèche.

### Exemple 3 : Induction de la Bêta Défensine-2 par les sucres d'avocat

### I. Ensemencement des cellules (JO) :

Des kératinocytes humains normaux sont ensemencés en plaque 96 puits (environ 20 000 cellules/puits), en présence d'un milieu spécifique enrichi en calcium (concentration finale 1,3 mM), tel que précédemment décrit dans la publication « Human β-Defensin-2 production in Keratinocytes is regulated by Interleukin-1, Bacteria, and the State of Differentiation », Alice Y. Liu et al., The Society for Investigative Dermatology, vol. 118, No. 2, Feb. 2002, pages 275 à 281.

### II. Traitement des cellules (J1) :

Après une incubation de 24h à 37°C, 5% CO₂ :
⇒ 2 rinçages avec 200 µl / puits de PBS (tampon phosphate en solution saline)
⇒ stimulation des cellules par 200 µl / puits (dans milieu supplémenté en Ca⁺⁺):
   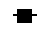 par l'extrait hydrosoluble de sucres d'avocat à des concentrations de 0,5, 0,05 et 0,005 % p/p de matière sèche
   ▪ par Il-1β à une concentration 100 ng/ml (contrôle positif d'induction de hBD-2)

### III. Fin du traitement (J2) : ELISA

Après 24h d'incubation, l'induction de l'hBD-2 est évaluée par une technique ELISA avec un anticorps spécifique (*goat polyclonal to human BD2 ; Abcam ; ab9871).* Les résultats obtenus avec l'extrait hydrosoluble de sucre d'avocat, lot A, contenant 40% de D-mannoheptulose et 40% de perséitol, par rapport à la matière sèche, sont résumés dans le tableau 9 suivant :

**Tableau 9**

| | Cellules contrôles | Témoin positif (IL-1β) | Lot A (0,005%) | Lot A (0,05%) | Lot A (0,5%) |
|---|---|---|---|---|---|
| hBD-2/MTT (DO) | 0,144 | 0,273 | 0,185 | 0,223 | 0,177 |
| hBD-2/MTT (DO) | 0,121 | 0,322 | 0,154 | 0,271 | 0,249 |
| hBD-2/MTT (DO) | 0,1 | 0,223 | 0,156 | 0,237 | 0,225 |
| hBD-2/MTT (DO) | 0,136 | 0,324 | 0,21 | 0,297 | 0,271 |
| Moyenne | 0,125 +/-0,017 | 0,285* +/-0,041 | 0,176* +/-0,023 | 0,257* +/-0,029 | 0,231* +/-0,035 |
| % d'augmentation vs contrôle | - | 128 | 41 | 105 | 84 |

| | | | | | |
|---|---|---|---|---|---|
| * Statistiquement significatif par rapport aux cellules contrôles (p<0,05 Test de Student) MTT : Methyl Thiazolyl Tetrazolium | | | | | |

On observe que, l'extrait hydrosoluble de sucres d'avocat, lot A, selon l'invention permet d'augmenter la quantité de hBD-2 produite.

Les résultats obtenus avec le lot B, contenant 10% de D-mannoheptulose et 70% de perséitol, par rapport à la matière sèche, sont résumés dans le tableau 10 suivant : DO= densité optique

**Tableau 10**

| | Cellules contrôles | Témoin positif (IL-1β) | lot B (0,005%) | lot B (0,05%) | lot B (0,5%) |
|---|---|---|---|---|---|
| hBD-2/MTT (DO) | 0,144 | 0,273 | 0,162 | 0,331 | 0,569 |
| hBD-2/MTT (DO) | 0,121 | 0,322 | 0,163 | 0,344 | 0,536 |
| hBD-2/MTT (DO) | 0,100 | 0,223 | 0,196 | 0,343 | 0,536 |
| hBD-2/MTT (DO) | 0,136 | 0,324 | 0,192 | 0,353 | 0,556 |
| Moyenne | 0,125 +/-0,017 | 0,285* +/-0,041 | 0,178* +/-0,016 | 0,343* +/-0,008 | 0,549* +/-0,014 |
| % d'augmentation/contrôle | - | 128 | 42 | 174 | 338 |

| | | | | | |
|---|---|---|---|---|---|
| * Statistiquement significatif par rapport aux cellules contrôles (p<0,05 Test de Student) | | | | | |

On observe que, d'une manière dose dépendante, l'extrait soluble de sucres d'avocat, lot B, permet d'augmenter la production d'hBD-2.

### Exemple 4 : effet des sucres d'avocat (lot A) sur l'expression de hBD-2 dans des cellules épithéliales.

### 1. Cellules

Les cellules KB (ATCC CCL-17), lignée de cellules épithéliales issues d'un carcinome oral humain, utilisées couramment dans les études sur la cavité buccale, ont été ensemencées en plaques 96 puits et cultivées dans du RPMI 1640 (Roswell Park Memorial Institute Medium) avec Glutamax™ I à 10% SVF (sérum de veau fœtal) + antibiotiques.

### 2. Traitement

Après 24 heures d'incubation, le milieu de culture a été éliminé et le tapis cellulaire a été rincé deux fois par du PBS.

Les cellules ont alors été traitées dans les conditions définies ci-dessous pendant 24 et 48 heures :
→ Cellules contrôles : milieu seul
→ TNFα (commercialisé par Sigma) à 100 ng/ml
→ extrait hydrosoluble de sucres d'avocat (40% mannoheptulose/40% perséitol) à 0,005-0,05 et 0,5% p/p (de matière sèche) (lot A)

3. Fin du traitement

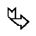 Dosage des peptides anti-microbiens par ELISA sur cellules

Après 48 heures d'incubation dans les différentes conditions de traitement, les β-défensines 2 et 3 ainsi que LL-37 présentes dans les cellules KB ont été dosées par une technique d'ELISA sur cellules.

Afin de déterminer le nombre de cellules totales dans chaque puits, un test au MTT a été réalisé en parallèle sur des cellules traitées dans les mêmes conditions.

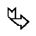 Exploitation des résultats

Pour chaque condition de traitement, la DO₄₅₀ PAMs (Peptides anti-microbiens) a été divisée par la DO₅₇₀ MTT pour obtenir la quantité de PAMs produite par cellule vivante.

Les moyennes et écart-types ont été calculés pour chaque condition et l'induction des différents PAMs a été calculée comme le pourcentage d'augmentation par rapport aux cellules contrôles.

### 4. Résultats

### Induction de HBD-2

Le TNF-α utilisé ici comme contrôle positif de l'induction des PAMs entraîne une augmentation significative de la production de hBD-2 par les cellules KB de 239% à 48h. Ce test valide le modèle.

L'extrait hydrosoluble de sucres d'avocat induit également une augmentation statistiquement significative de la production de hBD-2 dans les cellules KB (cf résultats tableau 11).

**Tableau 11**

| 48 heures | DO hBD2 / DO MTT | | | | | | | | Induction de HBD-2 | |
|---|---|---|---|---|---|---|---|---|---|---|
| | puits 1 | puits 2 | puits 3 | puits 4 | puits 5 | puits 6 | Moyenne | Ecart-type | *% d 'augmentation* | Significativité (test de Student) |
| témoin négatif | 0,000 | 0,000 | 0,000 | 0,019 | 0,047 | 0,000 | 0,011 | 0,017 | | |
| TNFα 100 ng/ml | 0,048 | 0,036 | 0,027 | 0,047 | 0,046 | 0,018 | 0,037 | 0,011 | *239* | p<0,05 |
| Lot A 0,005% | 0,052 | 0,004 | 0,031 | 0,058 | 0,038 | 0,012 | 0,032 | 0,020 | *198* | |
| Lot A 0,05% | 0,052 | 0,058 | 0,071 | 0,071 | 0,026 | 0,022 | 0,050 | 0,019 | *358* | p<0,05 |
| Lot A 0,5% | 0,174 | 0,200 | 0,147 | 0,176 | 0,142 | 0,135 | 0,162 | 0,023 | *1387* | p<0,01 |

### Induction de HBD-3

Le TNF-α utilisé ici comme contrôle positif de l'induction des PAMs entraîne une augmentation significative de la production de hBD-3 par les cellules KB de 30% à 48h. Ce test valide le modèle. L'extrait hydrosoluble de sucres d'avocat induit également une augmentation statistiquement significative de la production de hBD-3 dans les cellules KB (cf résultats tableau 12).

**Tableau 12**

| 48 heures | DO hBD3 / DO MTT | | | | | | | | Induction de HBD-3 | |
|---|---|---|---|---|---|---|---|---|---|---|
| | puits 1 | puits 2 | puits 3 | puits 4 | puits 5 | puits 6 | Moyenne | Ecart-type | *% d 'augmentation* | Significativité (test de Student) |
| témoin négatif | 0,342 | 0,423 | 0,275 | 0,271 | 0,293 | 0,283 | 0,315 | 0,054 | | |
| TNFα 100 ng/ml | 0,431 | 0,448 | 0,399 | 0,407 | 0,374 | 0,399 | 0,410 | 0,024 | *30* | p<0,01 |
| Lot A 0,005% | 0,448 | 0,432 | 0,338 | 0,423 | 0,321 | 0,329 | 0,382 | 0,053 | *21* | p<0,05 |
| Lot A 0,05% | 0,514 | 0,481 | 0,441 | 0,410 | 0,436 | 0,427 | 0,451 | 0,035 | *43* | p<0,01 |
| Lot A 0,5% | 0,451 | 0,492 | 0,436 | 0,429 | 0,417 | 0,489 | 0,452 | 0,029 | *44* | p<0,01 |

### Induction de LL-37

Le TNF-α utilisé ici comme contrôle positif de l'induction des PAMs entraîne une augmentation significative de la production de LL-37 par les cellules KB de 88% à 48h. Ce test valide le modèle. L'extrait hydrosoluble de sucres d'avocat induit également une augmentation statistiquement significative de la production de LL-37 dans les cellules KB. (cf résultats tableau 13)

**Tableau 13**

| 48 heures | DO LL-37 / DO MTT | | | | | | | | Induction de LL-37 | |
|---|---|---|---|---|---|---|---|---|---|---|
| | puits 1 | puits 2 | puits 3 | puits 4 | puits 5 | puits 6 | Moyenne | Ecart-type | *% augmentation* | Significativité (test de student) |
| témoin négatif | 0,033 | 0,047 | 0,031 | | 0,046 | 0,054 | 0,042 | 0,009 | | |
| TNFα 100 ng/ml | 0,080 | 0,072 | 0,077 | 0,091 | 0,082 | 0,076 | 0,080 | 0,006 | *88* | p<0,01 |
| Lot A 0,005% | 0,087 | 0,078 | 0,088 | 0,129 | 0,090 | 0,077 | 0,091 | 0,018 | *116* | p<0,01 |
| Lot A 0,05% | 0,098 | 0,094 | 0,097 | 0,103 | 0,091 | 0,063 | 0,091 | 0,013 | *115* | p<0,01 |
| Lot A 0,5% | 0,148 | 0,116 | 0,119 | | 0,173 | 0,119 | 0,135 | 0,022 | *219* | p<0,01 |

En conclusion, il a été mis en évidence que les sucres d'avocat (extrait hydrosoluble de sucres d'avocat) sont capables d'induire la synthèse des peptides anti-microbiens, et notamment de l'hBD-2, -3 et de LL-37 dans des cellules épithéliales de type KB.

### Exemple 5 : Effet des sucres d'avocat sur les molécules pro-inflammatoires

### Mode opératoire :

Des kératinocytes humains normaux sont ensemencés en plaque 24 puits (environ 50 000 cellules/puits), en présence d'un milieu spécifique enrichi en calcium (concentration finale 1,3 mM), tel que précédemment décrit dans la publication « Human β-Defensin-2 production in Keratinocytes is regulated by Interleukin-1, Bacteria, and the State of Differentiation », Alice Y. Liu et al., The Society for Investigative Dermatology, vol. 118, No. 2, Feb. 2002, pages 275 à 281.

Les cellules ont été traitées pendant 24h en présence de 0,05% p/p de sucres d'avocat (lot A, 40% mannoheptulose/40% perséitol). Les cytokines pro-inflammatoires IL-1β, IL-8 et TNF-α ont été dosées par une technique ELISA (kits R&D System) dans les surnageants de culture. La viabilité cellulaire est mesurée par un test au rouge neutre. Les résultats sont exprimés en fonction de la DO rouge neutre afin de ramener la quantité de cytokines produites à la quantité de cellules vivantes (tableaux 14, 15, 16).

### Résultats :

**Tableau 14**

| | IL1β (pg/ml) / DO rouge neutre | | | | |
|---|---|---|---|---|---|
| | puits 1 | puits 2 | puits 3 | Moyenne | Ecart-type |
| Cellules contrôles | 0,093 | 0,048 | 0,149 | 0,097 | 0,042 |
| Cellules + lot A à 0,05% MS | 0,058 | 0,009 | 0,012 | 0,026 | 0,022 |

**Tableau 15**

| | IL8 (pg/ml) / DO rouge neutre | | | | |
|---|---|---|---|---|---|
| | puits 1 | puits 2 | puits 3 | Moyenne | Ecart-type |
| Cellules contrôles | 506,624 | 402,282 | 907,228 | 605,378 | 217,649 |
| Cellules + lot A à 0,05% MS | 434,221 | 411,400 | 495,191 | 446,937 | 35,369 |

**Tableau 16**

| | TNFα (pg/ml) / DO rouge neutre | | | | |
|---|---|---|---|---|---|
| | puits 1 | puits 2 | puits 3 | Moyenne | Ecart-type |
| Cellules contrôles | 0,006 | 0,005 | 0,584 | 0,198 | 0,272 |
| Cellules + lot A à 0,05% MS | 1,515 | 0,511 | 0,916 | 0,980 | 0,413 |

Conclusion : Les sucres d'avocat (extrait hydrosoluble de sucres d'avocat) n'induisent pas la production des molécules pro-inflammatoires habituellement co-exprimées avec les béta-défensines (IL-1, IL-8, TNF-a). Ainsi, les sucres d'avocat sont des inducteurs des PAMs sans être pour autant pro-inflammatoires.

### Exemple 6 : Modulation de la synthèse de HBD-2 par les différents sucres présents dans l'extrait de sucres d'avocat

Le même test qu'à l'exemple 3 a été réalisé à partir de :
- un mélange de fructose (5%), glucose (5%) et saccharose (3%) ;
- D-mannoheptulose (40%) ;
- perséitol (40%) ;
- un mélange de fructose (5%), glucose (5%), saccharose (3%) et D-mannoheptulose (40%) ;
- un mélange de fructose (5%), glucose (5%), saccharose (3%) et perséitol (40%) ;
- un mélange de fructose (5%), glucose (5%), saccharose (3%), D-mannoheptulose (40%) et perséitol (40%) ; et
- un extrait de sucres d'avocat (lot A).

L'extrait des sucres d'avocat comprend 40 % en poids de D-mannoheptulose et 40 % en poids de perséitol, par rapport au poids total de la matière sèche.
Les résultas sont donnés dans le tableau 17 suivant :

**Tableau 17**

| Sucre(s) testé(s): | Modulation de la synthèse de HBD-2 |
|---|---|
| Proportions correspondant à 1% d'une solution à 5% de MS du lot A, soit 0,05% MS | (% augmentation par rapport aux cellules contrôles) |
| mélange fructose, glucose et saccharose | 0 |
| D-mannoheptulose | 31 |
| perséitol | 12 |
| mélange fructose, glucose, saccharose et D-mannoheptulose | 51 |
| mélange fructose, glucose, saccharose et perséitol | 37 |
| mélange fructose, glucose, saccharose, perséitol et D-mannoheptulose | 51 |
| sucres d'avocat | 49 |

Les sucres minoritaires de l'avocat (fructose, glucose et saccharose) n'ont pas d'activité sur la synthèse de HBD-2. A quantité égale (40% en poids, par rapport au poids de la matière sèche), le D-mannoheptulose est plus actif que le perséitol. Lorsque le D-mannoheptulose et/ou le perséitol est mélangé avec les sucres minoritaires de l'avocat (fructose, glucose et saccharose), on observe un effet de synergie.

Le mélange fructose, glucose, saccharose, perséitol et D-mannoheptulose (dit mélange reconstitué) et l'extrait hydrosoluble de sucres d'avocat obtenu à l'exemple 1 ont une activité équivalente.

### Exemple 7: Activité du D-mannoheptulose et du perséitol d'origine commerciale.

Le même test qu'à l'exemple 3 a été réalisé à partir de D-mannoheptulose et de perséitol disponibles dans le commerce. Les résultats sont donnés dans le tableau 18 suivant :

**Tableau 18**

| | Cellules contrôles | IL-1 beta Témoin positif | D-mannoheptulose (0,0012%) | D-mannoheptulose (0,012%) | Perseitol (0,0012%) | Perseitol (0,012%) |
|---|---|---|---|---|---|---|
| Moyenne | 0,040 | 0,104 | 0,089 | 0,089 | 0,063 | 0,090 |

Le D-mannoheptulose et le perséitol, testés séparément, sont capables d'induire la production d'hBD-2.

### Exemple 8: Effet des sucres d'avocat sur l'expression d'hBD-2 modulée par l'IL-4, dans le cadre de modélisation in vitro de la dermatite atopique

Des kératinocytes humains normaux sont ensemencés comme décrit dans l'exemple 3.

Après une incubation de 24h à 37°C, 5% de CO₂, le milieu de culture est éliminé et le tapis cellulaire rincé deux fois au PBS. Les cellules sont alors traitées pendant 24 heures dans les conditions définies ci-dessous :
- Cellules contrôles : milieu seul
- Il-1β à 100 ng/ml (contrôle positif d'induction d'hBD-2)
- Il-4 à 50 ng/ml
- Extrait hydrosoluble de sucres d'avocat (lot A) à 0,05% p/p de matière sèche
- Il-1β à 100 ng/ml et Il-4 à 50 ng/ml
- Il-4 à 50 ng/ml et extrait hydrosoluble de sucres d'avocat (lot A) à 0,05% p/p de matière sèche
- Il-1β à 100 ng/ml et Il-4 à 50 ng/ml et extrait hydrosoluble de sucres d'avocat (lot A) à 0,05% p/p de matière sèche

A la fin du traitement, l'induction d'hBD-2 est évaluée par une technique d'ELISA sur cellules, parallèlement, le nombre de cellules totales présentes dans chaque puits est déterminé par un test au MTT réalisé en parallèle sur des cellules traitées dans les mêmes conditions. Ainsi pour chaque condition, la DO₄₅₀ hBD-2 est divisée par la DO₅₇₀ MTT pour obtenir la quantité d'hBD-2 produite par cellule vivante.

Les résultats sont présentés dans le tableau 19 ci-dessous :

**Tableau 19**

| | Cellules contrôles | Il-1β (contrôle positif) | Il-4 | Lot A | Il-1β + Il-4 | Il-4 + Lot A | Il-1β + Il-4 + Lot A |
|---|---|---|---|---|---|---|---|
| hBD-2/MTT (DO) | 0,176 | 0,308 | 0,158 | 0,240 | 0,261 | 0,210 | 0,606 |
| hBD-2/MTT (DO) | 0,175 | 0,347 | 0,163 | 0,309 | 0,177 | 0,214 | 0,519 |
| hBD-2/MTT (DO) | 0,173 | 0,291 | 0,160 | 0,225 | 0,209 | 0,263 | 0,504 |
| Moyenne | 0,175 +/- 0,001 | 0,315** +/- 0,024 | 0,160 +/- 0,002 | 0,258* +/- 0,037 | 0,216*** +/- 0,035 | 0,229 +/- 0,024 | 0,543**** +/- 0,045 |

| | | | | | | | |
|---|---|---|---|---|---|---|---|
| * p<0,01 ; ** p<0,05 par rapport aux cellules contrôles (test T de Student) *** p<0,05 par rapport au contrôle positif (test T de Student) ****p<0,01 par rapport à Il-1β + Il-4 (test T de Student) | | | | | | | |

Le contrôle positif Il-1β induit une augmentation significative de la synthèse d'hBD-2 par rapport aux cellules contrôles (+80%).

De la même manière, l'extrait hydrosoluble de sucres d'avocat, lot A, provoque une augmentation de la synthèse d'hBD2 (+48%).

L'Il-4 seule n'a pas d'influence sur la quantité d'hBD-2 exprimée par les kératinocytes.

En présence d'Il-4, la synthèse d'hBD-2 induite par l'Il-1β est significativement inhibée de 32%.

Dans ces conditions, l'addition du lot A à Il-1β + Il-4 permet de ré-augmenter la synthèse d'hBD-2 : augmentation significative de 152% par rapport à Il-1β + Il-4.

### Conclusion :

La dermatite atopique est caractérisée par une déficience en peptides anti-microbiens (hBD-2, hBD-3, LL-37). Cette carence s'explique notamment par une dérégulation de la balance TH1/TH2 et une surproduction des cytokines TH2 (Il-4 et Il-13). Dans ce modèle, nous avons montré que l'extrait hydrosoluble de sucres d'avocat est capable de contrer l'inhibition d'hBD2 induite par l'Il-4. Les sucres d'avocat ont donc un intérêt dans la prise en charge de la dermatite atopique.

### Exemple 9 : formulations cosmétiques selon l'invention

**Crème anti acné n°1**

| | |
|---|---|
| Isononyl Isononanoate | 7,000 |
| Di-C₁₂₋₁₃ Alkyl Malate | 7,000 |
| Stéarate isocétyle | 5,000 |
| Butylène glycol | 3,000 |
| Oriza Sativa | 2,500 |
| **Extrait hydrosoluble de sucres d'avocat** | **3,000** |
| Dicaprylyle Ether | 2,000 |
| Salicylate de Silanediol | 2,000 |
| Alcool arachique | 1,650 |
| Trométhamine | 1,180 |
| Alcool cétylique | 1,000 |
| Acide salicylique | 1,000 |
| Glucoside ascorbyle | 1,000 |
| Glycine | 1,000 |
| Acétate de tocophéryle | 1,000 |
| Alcool béhénylique | 0,900 |
| Squalane | 0,790 |
| Citrate de Sodium | 0,660 |
| Copolymère PPG-12/SMDI | 0,500 |
| Glucoside Arachidyle | 0,450 |
| Parfum | 0,400 |
| Gomme sclerotium | 0,160 |
| Alcool cétéarylique | 0,130 |
| Acide citrique | 0,110 |
| Sepigel 305* | 0,100 |
| Système conservateur | QS |
| Eau | QSP 100 |

| | |
|---|---|
| **produit commercialisé par la société Seppic* | |

**Emulsion moussante lavante pour peaux acnéiques n° 1**

| | |
|---|---|
| Eau | QSP 100 |
| Arlatone duo* | 20,00000 |
| Glucoside de Coco- | 12,00000 |
| Guar hydroxypropyle | 2,00000 |
| **Extrait soluble de sucres d'avocat** | **1,00000** |
| Palmate de PEG-200 Glyceryl hydrogéné | 1,10000 |
| Cocoate de PEG-7 Glyceryl | 1,10000 |
| Salicylate de Silanediol | 1,00000 |
| Cocamide DEA | 1,00000 |
| Caprylyol Glycine | 0,50000 |
| Sorbate de Potassium | 0,50000 |
| Polyquatemium 10 | 0,40000 |
| Parfum | 0,40000 |
| Acide Citrique | 0,30000 |
| Zinc PCA | 0,20000 |

| | |
|---|---|
| **produit commercialisé par la société Quimasso* | |

**Emulsion moussante lavante pour peaux acnéiques n°2**

| | |
|---|---|
| Eau | QSP 100 |
| Arlatone duo* | 20,00000 |
| Coco-Glucoside | 12,00000 |
| Guar Hydroxypropyl | 2,00000 |
| **Extrait soluble de sucres d'avocat** | **2,00000** |
| Palmitate de PEG-200 Glyceryl hydrogéné | 1,10000 |
| Cocoate de PEG-7 Glyceryl | 1,10000 |
| Salicylate de Silanediol | 1,00000 |
| Cocamide DEA | 1,00000 |
| Glycine de Caprylyol | 0,50000 |
| Sorbate de Potassium | 0,50000 |
| Polyquatemium 10 | 0,40000 |
| Parfum | 0,40000 |
| Acide Citrique | 0,30000 |
| Zinc PCA | 0,20000 |

| | |
|---|---|
| **produit commercialisé par la société Quimasso* | |

**Pâte dentifrice**

| | |
|---|---|
| Eau | QSP 100 |
| Extrait soluble de sucres d'avocat | 2,00 |
| Monofuorophosphate de sodium | 0,75 |
| Fluorure de sodium | 0,10 |
| Sorbitol à 70% | 35 |
| Silice synthétique à fort pouvoir abrasif | 13 |
| Silice synthétique à faible pouvoir abrasif | 5 |
| Carboxymethylcellulose sodique | 1,6 |
| Lauryl sulfate de sodium | 1 |
| Arôme mentholé | 0,85 |
| Oxyde de titane | 0,5 |
| Lessive de soude | 0,5 |
| Cyclamate de sodium | 0,3 |
| Menthol | 0,15 |
| Saccharine sodique | 0,07 |

**Bain de bouche**

| | |
|---|---|
| ACTIMP 193 ® (peptides de lupin) | 2,00 |
| Cremophor RH40 ® | 0,30 |
| Glycérine | 15 |
| Saccharine sodique | 0,03 |
| Extrait soluble de sucres d'avocat | 1,00 |
| Arôme EUCA MINT | 0,08 |
| POBM | 0,20 |
| Sorbate de potassium | 0,50 |
| Eau | QSP100 |

## Revendications

1. Sucre, choisi parmi le D-mannoheptulose, le perséitol et leurs mélanges pour son utilisation dans le traitement ou la prévention de maladies liées à une modification de l'immunité innée et/ou acquise par augmentation de la production de peptides antimicrobiens de la famille des bétâ-défensines, avantageusement de la hBD-2, lesdites maladies étant choisies dans le groupe constitué par :
- les maladies liées à la présence de micro-organismes tels que les bactéries Gram+ et/ou Gram-, les champignons, les levures ou les virus ;
- les infections de la peau et des phanères, notamment choisies dans le groupe constitué par la folliculite, les pellicules, l'hyper-séborrhée, le furoncle, l'abcès, l'impétigo ou le panaris ;
- les dermatoses inflammatoires, telles que la dermatite atopique, l'eczéma de contact et/ou atopique, le psoriasis, l'acné, les dermites irritatives ;
- les brûlures ;
- les pathologies liées à un déficit de la barrière cutanée, telles que les peaux hyper-réactives, atopiques, , ou des pathologies liées à des peaux fragilisées par une agression environnementale ;
- les maladies parodontales ;
- les pathologies articulaires inflammatoires telles que l'arthrose ;
- les infections des muqueuses, notamment des muqueuses vaginale, intestinale, respiratoire, nasale ou auriculaire ;
- les infections du système oculaire ;
ou pour favoriser la cicatrisation, dans les processus de cicatrisation normaux ou pathologiques, tels que les ulcères et les escarres ;
ou pour la protection des peaux :
- pathologiques immatures des nourrissons ou des enfants ; ou
- pathologiques des individus adultes ou âgés ;
ledit sucre est formulé avec un excipient approprié pharmaceutiquement acceptable en une composition qui est un médicament ou une composition vétérinaire.

2. Sucre pour son utilisation selon la revendication 1, **caractérisé en ce que** la composition comprend 0,001 à 30 % en poids de D-mannoheptulose, par rapport au poids total de ladite composition, et/ ou 0,001 à 30 % en poids de perséitol, par rapport au poids total de ladite composition.

3. Sucre pour son utilisation selon l'une quelconque des revendications précédentes, **caractérisé en ce que** la source de D-mannoheptulose et/ou de perséitol est un extrait hydrosoluble de sucres d'avocat, avantageusement susceptible d'être obtenu par un procédé comprenant les étapes successives suivantes :
- obtention d'un tourteau d'avocat, avantageusement du fruit de l'avocat, par séchage de l'avocat puis extraction des lipides ; ensuite
- cryobroyage et délipidation complète dudit tourteau, puis décantation et centrifugation afin de récupérer une fraction soluble riche en sucres en C7 (élimination du gâteau) ;
- déminéralisation sur résine ionique de ladite fraction soluble, obtenue à l'étape précédente ; puis
- ultrafiltration à 10 000 daltons ; et
- concentration sous vide et conditionnement.

4. Sucre pour son utilisation selon la revendication 3, **caractérisé en ce que** l'extrait hydrosoluble de sucres d'avocat comprend en poids, par rapport au poids total de la matière sèche de l'extrait (composition relative déterminée par HPLC):
| | |
|---|---|
| - D-mannoheptulose | 5 à 80 % |
| - Perséitol | 5 à 80 % |
| - Saccharose | inférieur à 10% |
| - Glucose | inférieur à 10% |
| - Fructose | inférieur à 10% |

5. Sucre pour son utilisation selon l'une quelconque des revendications précédentes, **caractérisé en ce que** la composition comprend en outre un extrait peptidique d'avocat.

6. Composition cosmétique comprenant 0,001 à 30% en poids de D-mannoheptulose, par rapport au poids total de ladite composition, et 0,001 à 30% en poids de perséitol, par rapport au poids total de ladite composition pour son utilisation dans le traitement des peaux et/ou des muqueuses sensibles, irritées, sèches, âgées, intolérantes, présentant un trouble de la barrière cutanée, fragilisées par une agression environnementale présentant des rougeurs cutanées ou présentant un déséquilibre immunologique non pathologique, par application de la composition sur la peau et/ou les muqueuses.

7. Composition pour son utilisation selon la revendication 6, **caractérisée en ce que** la source de D-mannoheptulose et/ou de perséitol est un extrait hydrosoluble de sucres d'avocat, avantageusement susceptible d'être obtenu par un procédé comprenant les étapes successives suivantes :
- obtention d'un tourteau d'avocat, avantageusement du fruit de l'avocat, par séchage de l'avocat puis extraction des lipides ; ensuite
- cryobroyage et délipidation complète dudit tourteau, puis décantation et centrifugation afin de récupérer une fraction soluble riche en sucres en C7 (élimination du gâteau) ;
- déminéralisation sur résine ionique de ladite fraction soluble, obtenue à l'étape précédente ; puis
- ultrafiltration à 10 000 daltons ; et
- concentration sous vide et conditionnement.

8. Composition pour son utilisation selon la revendication 6 ou 7, comprenant en outre un extrait peptidique d'avocat.

## Patentansprüche

1. Zucker, ausgewählt aus der D-Mannoheptulose, dem Perseitol und deren Gemischen für seine Verwendung für die Behandlung oder die Vorbeugung von Krankheiten, die mit einer Veränderung der angeborenen und/oder erworbenen Immunität durch Erhöhung der Produktion von antimikrobiellen Peptiden aus der Familie der Beta-Defensine, in vorteilhafter Weise von hBD-2, verbunden sind, wobei die Krankheiten aus der Gruppe ausgewählt sind, die gebildet ist von:
- den Krankheiten, die mit der Anwesenheit von Mikroorganismen wie den Gram+ und/oder Gram- Bakterien, den Pilzen, den Hefen oder den Viren verbunden sind;
- den Infektionen der Haut und der Hautanhangsgebilde, insbesondere ausgewählt aus der Gruppe, die von der Follikulitis, den Schuppen, der Hyperseborrhoe, dem Furunkel, dem Abszess, der Impetigo oder des Panaritiums gebildet ist;
- den entzündlichen Dermatosen wie der atopischen Dermatitis, dem Kontakt- und/oder atopischen Ekzem, der Schuppenflechte, der Akne, den irritativen Dermatiden;
- den Verbrennungen;
- den Erkrankungen, die mit einem Defizit der Hautbarriere verbunden sind, wie die hyperreaktive, atopische Haut, oder Pathologien, die mit der durch eine Umweltaggression geschwächten Haut verbunden sind;
- den Parodontalkrankheiten;
- den entzündlichen Gelenkerkrankungen wie die Arthrose;
- den Infektionen der Schleimhäute, insbesondere der Schleimhäute der Vagina, des Darms, der Atemwege, der Nase oder des Gehörgangs;
- den Infektionen des okularen Systems;
oder um die Wundheilung zu fördern, innerhalb von normalen oder pathologischen Wundheilungsprozessen, wie bei den Geschwüren oder den Druckgeschwüren;
oder für den Schutz der Haut:
- Unreifeerkrankungen der Säuglinge oder der Kinder; oder
- Erkrankungen von erwachsenen oder alten Individuen;
wobei der Zucker mit einem pharmazeutisch akzeptablen, geeigneten Hilfsstoff in einer Zusammensetzung formuliert ist, die ein Arzneimittel oder eine Veterinärzusammensetzung ist.

2. Zucker für seine Verwendung nach Anspruch 1, **dadurch gekennzeichnet, dass** die Zusammensetzung 0,001 bis 30 Gew.-% D-Mannoheptulose in Bezug auf das Gesamtgewicht der Zusammensetzung und/oder 0,001 bis 30 Gew.-% Perseitol in Bezug auf das Gesamtgewicht der Zusammensetzung umfasst.

3. Zucker für seine Verwendung nach einem der vorangehenden Ansprüche, **dadurch gekennzeichnet, dass** die Quelle von D-Mannoheptulose und/oder von Perseitol ein wasserlöslicher Extrakt von Avocadozuckern ist, der in vorteilhafter Weise imstande ist, durch ein Verfahren gewonnen zu werden, das die folgenden aufeinanderfolgenden Schritte umfasst:
- Gewinnen eines Avocadoölkuchens, in vorteilhafter Weise von der Avocadofrucht, durch Trocknen der Avocado, gefolgt von der Extraktion der Lipide; danach
- Gefrierzerkleinern und vollständiges Delipidieren des Ölkuchens, danach Abscheiden und Zentrifugieren, um eine lösliche Fraktion reich an C7-Zuckern zu gewinnen (Eliminieren des Kuchens);
- Demineralisieren der löslichen Fraktion, die in vorangehendem Schritt erhalten wurde, auf ionischem Harz, dann
- Ultrafiltration bei 10.000 Dalton; und
- Konzentrieren im Vakuum und Verpacken.

4. Zucker für seine Verwendung nach Anspruch 3, **dadurch gekennzeichnet, dass** der wasserlösliche Extrakt von Avocadozuckern gewichtsanteilmäßig in Bezug auf das Gesamtgewicht der Trockenmasse des Extrakts (relative Zusammensetzung, bestimmt durch HPLC) umfasst:
| | |
|---|---|
| - D-Mannoheptulose | 5 bis 80% |
| - Perseitol | 5 bis 80% |
| - Saccharose | unter 10% |
| - Glucose | unter 10% |
| - Fructose | unter 10%. |

5. Zucker für seine Verwendung nach einem der vorangehenden Ansprüche, **dadurch gekennzeichnet, dass** die Zusammensetzung ferner einen Avocado-Peptidextrakt umfasst.

6. Kosmetische Zusammensetzung, umfassend 0,001 bis 30 Gew.-% D-Mannoheptulose in Bezug auf das Gesamtgewicht der Zusammensetzung, und 0,001 bis 30 Gew.-% Perseitol in Bezug auf das Gesamtgewicht der Zusammensetzung für ihre Verwendung bei der Behandlung sensibler, irritierter, trockener, alter, intoleranter Haut und Schleimhäute, die eine Störung der Hautbarriere aufweisen, durch eine Umweltaggression geschwächt sind, aufweisend Hautrötungen oder aufweisend ein nicht pathologisches immunologisches Ungleichgewicht, durch Anwendung der Zusammensetzung auf der Haut und/oder den Schleimhäuten.

7. Zusammensetzung für ihre Verwendung nach Anspruch 6, **dadurch gekennzeichnet, dass** die Quelle von D-Mannoheptulose und/oder von Perseitol ein wasserlöslicher Extrakt von Avocadozuckern ist, der in vorteilhafter Weise imstande ist, durch ein Verfahren gewonnen zu werden, das die folgenden aufeinanderfolgenden Schritte umfasst:
- Gewinnen eines Avocadoölkuchens, in vorteilhafter Weise von der Avocadofrucht, durch Trocknen der Avocado, gefolgt von der Extraktion der Lipide; danach
- Gefrierzerkleinern und vollständiges Delipidieren des Ölkuchens, danach Abscheiden und Zentrifugieren, um eine lösliche Fraktion reich an C7-Zuckern zu gewinnen (Eliminieren des Kuchens);
- Demineralisieren der löslichen Fraktion, die in vorangehendem Schritt erhalten wurde, auf ionischem Harz; dann
- Ultrafiltration bei 10.000 Dalton; und
- Konzentrieren im Vakuum und Verpacken.

8. Zusammensetzung für ihre Verwendung nach Anspruch 6 oder 7, umfassend ferner einen Avocado-Peptidextrakt.

## Claims

1. A sugar selected in the group consisting of D-mannoheptulose, perseitol and mixtures thereof for use in the treatment and/or prevention of diseases related to a modification of the innate and/or acquired immunity by increasing the production of antimicrobial peptides of the beta-defensin family, advantageously hBD-2, said diseases being selected from the group consisting of:
- diseases related to the presence of microorganisms such as Gram+ and/or Gram- bacteria, fungi, yeasts or viruses;
- infections of the skin and cutaneous appendages, in particular those selected from the group consisting of folliculitis, dandruff, hyper-seborrhoea, boils, abscesses, impetigo or panaris;
- inflammatory dermatoses, such as atopic dermatitis, contact and/or atopic eczema, psoriasis, acne and irritant contact dermatitis;
- burns;
- pathologies related to a deficit of the skin barrier, such as hyper-reactive, atopic skins, or pathologies related to skins weakened by an environmental attack;
- parodontal diseases,
- inflammatory articular pathologies such as arthritis;
- infections of the mucous membranes, in particular the vaginal, intestinal, respiratory, nasal or auricular mucosa;
- infections of the ocular system;
or for enhancing the healing processes in normal or pathological cicatrisation, such as ulcers and pressure ulcers;
or for the protection of:
- pathological, immature skins of babies and children; or
- pathological skins of adult or elderly individuals;
said sugar is formulated with a suitable pharmaceutically acceptable excipient into a composition which is a veterinary medication or composition.

2. The sugar for use according to claim 1, **characterized in that** the composition comprises 0.001 to 30 wt.% of D-mannoheptulose, based on the total weight of said composition, and/or 0.001 to 30 wt.% of perseitol, based on the total weight of said composition.

3. The sugar for use according to any one of the preceding claims, **characterised in that** the source of D-mannoheptulose and/or perseitol is a hydrosoluble extract of sugars of avocado, advantageously obtainable by a process comprising the following successive steps:
- obtaining an avocado oil cake, advantageously from the avocado fruit, by drying the avocado then extracting the lipids; then
- cryogenic grinding and total delipidation of said oil cake, then decanting and centrifuging so as to recover the soluble fraction rich in C7 sugars (elimination of the cake);
- demineralisation over an ionic resin of said soluble fraction obtained from the preceding step; then
- ultrafiltration at 10 000 daltons; and
- concentration under vacuum and packaging.

4. The sugar for use according to claim 3, **characterised in that** the hydrosoluble extract of sugars of avocado comprises per weight, based on the total weight of the dry matter in the extract (relative composition determined by HPLC):
| | |
|---|---|
| - D-mannoheptulose | 5 to 80% |
| - Perseitol | 5 to 80% |
| - Saccharose | less than 10% |
| - Glucose | less than 10% |
| - Fructose | less than 10%. |

5. The sugar for use according to any one of the preceding claims, **characterised in that** the composition further comprises a peptidic extract of avocado.

6. A cosmetic composition comprising 0.001 to 30 wt.% of D-mannoheptulose, based on the total weight of said composition, and 0.001 to 30 wt.% of perseitol, based on the total weight of said composition for treating sensitive, irritated, dry, aged, intolerant skins and/or mucosa, skins and/or mucosa having a skin barrier disorder, weakened by an environmental attack having skin redness or having a non-pathological immunologic imbalance, by application of the composition on the skin and/or mucosa.

7. The composition for use according to claim 6, **characterized in that** the source of D-mannoheptulose and/or perseitol is a hydrosoluble extract of sugars of avocado, advantageously obtainable by a process comprising the following successive steps:
- obtaining an avocado oil cake, advantageously from the avocado fruit, by drying the avocado then extracting the lipids; then
- cryogenic grinding and total delipidation of said oil cake, then decanting and centrifuging so as to recover the soluble fraction rich in C7 sugars (elimination of the cake);
- demineralisation over an ionic resin of said soluble fraction obtained from the preceding step; then
- ultrafiltration at 10 000 daltons; and
- concentration under vacuum and packaging.

8. The composition for use according to claim 6 or 7, further comprising a peptidic extract of avocado.
